Europäisches Patentamt

European Patent Office

Office européen des brevets

(11) Publication number: **0 104 178**
**B1**

## (12) EUROPEAN PATENT SPECIFICATION

(45) Date of publication of patent specification: **10.07.85**

(51) Int. Cl.⁴: **C 12 P 7/06**

(21) Application number: **82903357.0**

(22) Date of filing: **05.11.82**

(86) International application number:
**PCT/SE82/00369**

(87) International publication number:
**WO 83/01627 11.05.83 Gazette 83/11**

(54) **A PROCESS FOR THE PRODUCTION OF ETHANOL.**

(30) Priority: **06.11.81 SE 8106611**

(43) Date of publication of application:
**04.04.84 Bulletin 84/14**

(45) Publication of the grant of the patent:
**10.07.85 Bulletin 85/28**

(84) Designated Contracting States:
**FR**

(56) References cited:
**DE-A-2 919 518**
**SE-A-78 011 335**
**SE-A-79 017 380**
**SE-B- 416 315**

(73) Proprietor: **ALFA-LAVAL AB**
**Box 500**
**S-147 00 Tumba (SE)**

(72) Inventor: **WALLNER, Mats**
**Östgötagatan 81 4 tr**
**S-116 64 Stockholm (SE)**

(74) Representative: **Burman, Tore et al**
**Bergling & Sundbergh AB P.O. Box 7645**
**S-103 94 Stockholm (SE)**

Courier Press, Leamington Spa, England.

## Description

The present invention relates to a process for the production of ethanol by continuous fermentation of a carbohydrate containing substrate of a raw material, which in addition to fermentable carbohydrates also contains cellulose fibres and/or other non-fermentable solid material. More specifically the invention relates to a further development of applicant's preceding patented processes for continuous ethanol fermentation at preferably atmosphere pressure in a fermentor with continuous yeast recirculation and continuous stillage recirculation.

The Swedish patent application 7801133-5 (also GB—A—2013716 and DE—A—2903273) discloses a method for continuous ethanol fermentation preferably at atmospheric pressure with continuous yeast recirculation and continuous stillage recirculation. The most important advantages of this method are the possibility to ferment a substrate containing a high concentration of fermentable substances, the addition of a minimum amount of water to the process, thereby minimizing the energy requirement for distillative separation of the ethanol, and the production of a stillage of such a high concentration that the stillage, previously constituting a waste disposal problem, now offers an essential positive contribution to the total process economy, for example as a raw material for animal feed stuff of high quality. Since the discharge of a concentrated stillage and a minimum water input according to this process is as a result of recirculating to the fermentor large amounts of stillage of the same concentration as the discharged stillage and the establishment of a high equilibrium concentration of non-fermentable substance in the fermenter, the success of the method can largely be derived from the surprising discovery that conventional yeast species were adaptable to give sufficient ethanol productivity despite the high concentration of salts and other dissolved non-fermentable material in the fermenter.

According to the Swedish patent application 7901738-0 (GB—A—2044799 and DE—A—2903273) the distillation step in the continuous ethanol process was modified so that the discharge of a further concentrated stillage was made possible. The distillation step for separating ethanol from the yeast-free liquor was divided into an evaporator section and a stripping section, the stillage stream recirculated in the process was discharged from the bottom of the evaporator section, that is from an intermediate distillation level in the distillation step, while the stillage to be discharged was withdrawn from the bottom of the stripping section. Among process improvements obtained through this modification, the most important advantage is that the concentrated stillage stream can be discharged from the distillation step at the same time as there is obtained through recirculation of a stillage stream of lower dry substance content than the discharged stillage stream a lower equilibrium level as to the concentration of non-fermentable substance in the fermenter compared with the above said original process at the same process water input.

Ethanol can be produced from several different raw materials of vegetable origin, which either contain directly fermentable sugars or polysaccharides which through enzymatic degradation or other degradation procedures can be converted to fermentable sugar. In addition to fermentable substance and soluble non-fermentable substance, these raw materials contain various amounts of non-fermentable solid constituents such as fibres, shells, lignin etc. On hydrolysis and fermentation of starch raw material such as grain, these solid substances occur in amounts that would cause clogging problems in the continuous fermentation circuit, for example clogging of the nozzle openings of the nozzle centrifuges suitably employed for the yeast separation. Besides, the viscosity of the concentrated stillage streams is negatively effected and may limit the possibility to reach maximum dry substance content in the discharged stillage.

According to a previous Swedish patent application 7811826-2 (SE—416315 also DE—A—2946161 and CA—1110986) this problem is solved by removing the fibres and other solid material directly from the raw material stream by straining and washing out remaining fermentable material from the solids by means of recirculated stillage. A disadvantage of this process, however, is that despite the use of a counter-current multi step washing requiring a relatively costly equipment, a complete recovery of the remaining fermentable substance from the solid material is not obtained.

According to the present invention there is disclosed a process, which is characterized in that the solid material is fed to the fermenter together with a substrate and that the solid material is separated from the stream of fermentation liquor withdrawn from the fermentor before the fermentation liquor is sent to the yeast separation step and the successive ethanol separation step, the solid substance being discharged from the fermentation circuit.

By this process the solid material is prevented reaching the yeast separater and the distillation plant, at the same time the content of fermentable material in the solids is very low since the concentration of fermentable material in the fermentor is maintained at a very low level, usually less than 0.5% by weight. The removal of the solid material can be carried out by any straining device or similar equipment. Since the stream of fermentation liquor, which due to an ethanol content in the range of 3—7% by weight must necessarily be large, a substantial part of the fermentation liquor will accompany the fibre phase if a rapid and efficient straining is to be carried out for example by means of a bow sieve. The liquor that accompanies the solid material will thus contain considerable amounts of yeast and ethanol.

According to a preferred embodiment of the invention, the rejected solids from said draining step are therefore washed with recirculated stillage in such an amount that a dilution of about 3—4 times is obtained. Then the wash liquor is separated off from the solid material in a suitable device, such as a centrifugal sieve, and is returned to the fermenter. Thereby the major parts of the remaining yeast and ethanol are washed out.

For the washing step also part of or all of the required process water input can be used. If for example the raw material is a grain product, the process water is usually added in the so called "mashing in step" and also in the form of direct steam in a successive hydrolysis step. Since this process water wholly or partly can be replaced by recirculated stillage, it is realized that the input of water to other parts of the process, in the present case to the washing step, will not effect on the total amount of water fed to the process.

The solid substance stream strained off after the washing step and having a dry substance content in the range of 25—35% by weight will still contain a small amount of ethanol. Therefore, as a further preferred embodiment of the invention the strained solid stream is subjected to further dewatering, for example by means of a press device, whereby something like half of the remaining ethanol can be pressed out with the press water and returned to the fermenter, while a press mass of a dry substance content of about 40% can be discharged from the process.

By discharging the solid material from the process circuit before the yeast separation step and the distillation step, the possibility to achieve a maximum concentration in the stillage discharged from the distillation step are improved. If the distillation step is designed according to the above said Swedish patent application 7901738-0 that is taking out a stillage stream at an intermediate level for recirculation and discharging stillage from the bottom of a stripping section, the present invention makes possible the production of a still more concentrated stillage stream from the stripper than before. If the solid material is passed through the yeast separating step or is drained off prior to the yeast separation step and rejoined with the feed to the stripper, the fluidity may be reduced in the bottom of the stripper so that the maximum upper dry maximum concentration of non-fermentable material in the fermenter cannot be fully utilized. Therefore, by separating off the solid material between the fermenter and the yeast separation step according to the invention, the limit for the dry substance content in the discharged stillage can be further raised to the range of 35—45% by weight and the process water input can be further reduced.

For efficient recovery of all waste products at the process according to the invention, it is further suggested that the concentrated stillage from the distillation step is combined with the removed solid material, whereafter the mixture is dried in suitably an indirect heated drying plant. The steam driven off in the drying plant is reused in the process for heating purpose such as heating a stripping unit or a succeeding rectifying unit, wherein thermocompression can also be used to raise the energy level of the steam. Further examples of reusing the drying steam are as a heat source and process water in the substrate preparation step. As an alternative to thermo-compression, the drying can be carried out under overpressure so that drying steam of higher energy level is obtained directly.

The invention will now be further described by means of a preferred embodiment of the same, wherein reference is made to the accompanied drawing showing a flow chart of a continuous ethanol fermentation process according to the invention.

A stream of raw material in 1 of suitable fine divided form, such as crushed corn, is fed together with a stream 3 of pure water and a stream 4 of recirculated stillage from the distillation step 5 to a substrate preparation step 2 comprising liquifaction and saccharification. From the substrate preparation step 2 the substrate stream 6 is fed into the fermenter 7, in which steady-state concentrations are maintained, the ethanol concentration being in the range of 3—7% by weight and the concentration of fermentable substance being low, preferably less than 0.5% by weight. From the fermentor a carbon dioxide stream 8 is continuously discharged.

From the fermenter a stream of fermentation liquor 9 is withdrawn. From the stream 9 fibres and other solid material are strained off in a strain device 10, from which a stream 11 of rejected material and a stream 12 of solid impoverished material are discharged. The stream 12 of fermentation liquor is conveyed to a centrifugal separater 13, from which there is discharged a heavier yeast suspension phase 14 which is recirculated to the fermenter 7 and a lighter essentially yeast-free phase 15 which is conveyed to the distillation unit 5. Before that the yeast-free stream 15 is subjected to heat exchange in a heat exchanger 16 with a recirculated stillage stream 17 from the distillation unit 5.

The distillation unit 5 is divided into an evaporator unit 18 and a stripping unit 19. The yeast-free stream of fermentation liquor 15 is fed to the evaporator unit 18, from the top of which discharges ethanol enriched vapours which may be conveyed to an optionally succeeding rectifying step. From the bottom of the evaporator unit 18, representing an intermediate distillation level in the distillation unit 5, a stillage stream 20 is discharged, a larger part of which is recirculated in the form of said stillage stream 17 to the fermentation circuit and a smaller part 21 is fed to the stripping unit 19. From the stripping unit 19, which is indirectly heated through 22, an overhead vapour stream 23 is discharged, which also contains the remaining ethanol of the stillage stream 21. The vapour stream 23 is fed to the evaporator unit 18 as direct heating medium. From the bottom of the stripping unit 19 a concentrated stillage stream 14 is discharged, the concentration of which, due to the elimination of solid material before the distillation step according to the invention, may reach a dry substance content exceeding 30% by weight, in some cases even exceeding 35% by weight. The reject stream 11 from the straining device 10 is conveyed to a further straining step 25 being for example a centrifugal sieve device, in which the reject stream is simultaneously diluted and washed with a stream 26, which is part of the recirculated stillage stream 17 after having been subjected to cooling in heat exchanger 16 and optionally further cooling in heat exchanger 27. In the straining device 25 a major part of the washing liquor is separated off and returned through 29 to the fermenter 7. The solid material strained off and having a dry substance content of for example the range 25—35% by weight, is conveyed through 30 to a press device 31, in which a large part of the remaining liquor is pressed off and returned through 32 to the fermenter. The remaining press mass, now having a dry substance content of about 40% by weight or higher, is discharged through 33, mixed with the stillage stream 24 and fed to a drying plant 34. From the drying plant 34 a dried product 35 is discharged, which can be utilized as animal feed stuff and having a protein content which may amount to about 30% by weight.

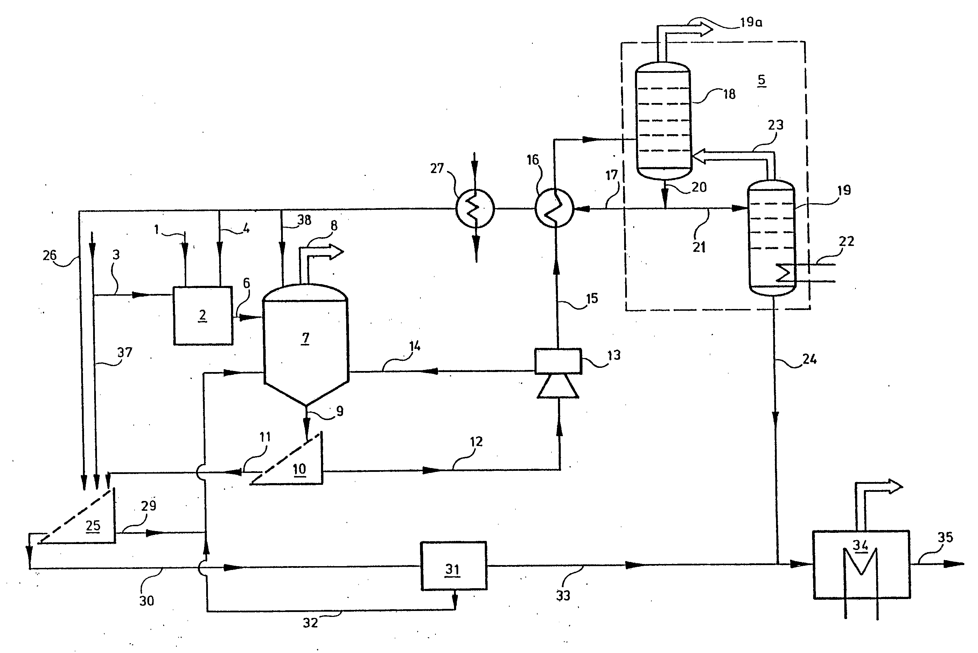

As alternative washing liquor at the straining step 25 pure water supplied to the process can also be used, shown in the figure by line 37. From the figure it can further be seen that the recirculated stillage stream 17 can be returned at different points and in various portions in the process circuit. As to stillage recirculation, in addition to said streams 26, i.e. washing liquor at the straining step 25, and the stream 4 for substrate preparation, stillage can of course be recirculated directly to the fermenter 7, shown by line 38.

In the specification and patent claims the term "solid substance" has been used for solid material of such a size that permits straining off by means of conventional straining devices. In this context it should be mentioned that distinct limits can hardly be drawn between particulate or fibrous substances, suspended finer material, colloidal material etc. The recirculated yeast fraction contains for example suspended yeast cells, and the other "yeast-free" and streams poor in "solid material" contain various amounts of i.e. fine suspended and colloidal proteins. Besides these protein aggregates are subjected to a certain modification when passing through the distillation step. By being subjected to heat therein, coagulation occurs and larger aggregates are formed. When these larger aggregates through the recirculated stillage stream once again reach the straining step 10 and 25, their tendency to stay in the reject phase has increased, and consequently the process according to the invention also implies that the stripping unit is relieved from part of these viscosity increasing aggregates, while still these desirable proteins for feeding purpose are recovered through remixing the sieve reject with stillage concentrate from the stripping unit.

## Claims

1. A process for the production of ethanol by continuous fermentation of a carbohydrate containing substrate (6) of a raw material which contains fermentable substances as well as non-fermentable soluble and solid substances, wherein the substrate after optionally having been treated in a substrate preparation step (2) comprising for example hydrolysis of the raw material, is fermented in a fermenter (7), comprised in a continuous process circuit including the fermenter (7), a yeast separation step (13) and a distillation step (5), a stream (9) of fermentation liquor is continuously withdrawn from the fermenter (7) and separated in said yesat separation step (13) into a yeast concentrate stream (14), which is continuously recirculated to the fermenter to maintain in the fermenter a concentration of active yeast cells corresponding to a certain ethanol productivity, and into an essentially yeast free stream (15), said yeast free stream (15) is continuously separated in said distillation step into an ethanol enriched stream (19a), which is discharged from the process circuit, and into at least one stillage stream (24, 17), part of said stillage stream (17) being continuously recirculated to the fermenter and/or to said substrate preparation step and the remaining part of said stillage stream (24) being discharged from the process circuit, characterized in that the substrate fed to the fermenter contains said solid substance, and that said stream of fermentation liquor (9) is separated into a stream (11) enriched in solid substance and into a solid impoverished stream (12) which is fed to said yeast separation step (13), that said stream (11) enriched in solid substance is washed by a stillage stream (26) which is recirculated in the process circuit and having been cooled, e.g. through heat exchange with said yeast-free flow (15), to a temperature not harmful to the yeast cells which remain in said stream enriched in solid substance, and/or with part of the required water input (37) to the process circuit, whereafter the washed solid substance is separated e.g. in a straining step (25) into an essentialy yeast-free solid substance stream (30), which is discharged, and a wash liquor stream (29), which is returned to the fermentor.

2. A process according to claim 1, characterized in that said stream enriched in solid substance (11) is washed with at least part of a first stillage stream (17), which is withdrawn from an intermediate distillation level in the distillation step (5) and recirculated into said process circuit, and that a second stillage stream (24) of higher dry substance than said first stillage stream (17) is discharged as a bottom product from said distillation step (5) and removed from said process circuit.

3. A process according to any of the preceding claims, characterized in that said yeast-free solid substance stream (30) is subjected to further concentration, e.g. in a pressing step (31), thereby being separated into a concentrate stream (33) and a liquor stream (32) which is returned to said process circuit, suitably to the fermenter (7).

4. A process according to any of the preceding claims, characterized in that a first stillage stream (17) is discharged at an intermediate distillation level in the distillation step (5) and recirculated in said process, and that a second stillage stream (24) with a dry substance content of more than 35% by weight is discharged as bottom product from said distillation step (5) and is removed from said process circuit.

5. A process according to any of the preceding claims, characterized in that said yeast-free solid substance stream (30) or said concentrate stream (33) is dried in a drying step (34) together with a stillage stream (24) discharged as a bottom product in the distillation step (5) to obtain a dried product (35) usable as a feed stuff.

6. A process according to claim 5, characterized in that steam produced in the drying step (34) is used after optional thermo-compression as a heating medium in said process circuit, for example as heating medium in the distillation step (5) and/or in the substrate preparation step (2).

**Patentansprüche**

1. Verfahren zur Herstellung von Äthanol durch kontinuierliche Fermentation eines kohlehydrathaltigen Substrats (6) aus einem Rohstoff, der fermentierbare Substanzen sowie nichtfermentierbare lösliche und feste Substanzen enthält, indem man das Substrat gegebenenfalls nach der Behandlung in einem Substrat-Vorbereitungsschritt (2), beispielsweise einer Hydrolyse des Rohstoffs, in einem Fermenter (7) fermentiert, der sich in einem kontinuierlichen Prozesskreis befindet, der den Fermenter (7), einen Hefeabtrennschritt (13) und einen Destillationsschritt (5) enthält, einen Strom (9) Fermentationsflüssigkeit kontinuierlich vom Fermenter (7) abnimmt und im Hefeabtrennschritt (13) zu einem Hefekonzentratstrom (14), den man kontinuierlich zum Fermenter zurückführt, um in diesem eine einer bestimmten Äthanolproduktivität entsprechende Konzentration aktiver Hefezellen aufrechtzuerhalten, und einem im wesentlichen hefefreien Strom (15) auftrennt, den man im Destillationsschritt kontinuierlich zu einem äthanolreichen Strom (19a), den man aus dem Prozesskreis abführt, und mindestens einem Schlämpestrom (24, 17) aufteilt, und einen Teil (17) der Schlämpe kontinuierlich zum Fermenter und/oder dem Substrat-Vorbereitungsschritt zurückführt, während man den übrigen Teil (24) der Schlämpe aus dem Prozess abführt, dadurch gekennzeichnet, dass das dem Fermenter zugeführte Substrat die Festsubstanz enthält und dass der Strom (9) der Fermentationsflüssigkeit zu einem feststoffreichen Strom (11) und einem feststoffarmen Strom (12) aufgetrennt wird, den man dem Hefeabtrennschritt (13) zuführt, dass man den feststoffreichen Strom (11) mit einer im Prozesskreis rückgeführten Schlämpe (26), die beispielsweise durch Wärmeaustausch mit dem hefefreien Strom (15) auf eine für die im feststoffreichen Strom verbeibenden Hefezellen nicht schädliche Temperatur gekühlt worden ist, und/oder mit einem Teil des für den Prozess erforderlichen Wassers (37) wäscht, wonach man die gewaschene Festsubstanz beispielsweise in einem Siebschritt (25) zu einem im wesentlichen hefefreiem Feststoff (30), den man abführt, und einem Waschflüssigkeitsstrom (29) auftrennt, den man zum Fermenter zurückführt.

2. Verfahren nach Anspruch 1, dadurch gekennzeichnet, dass der feststoffreiche Strom (11) mit mindestens einem Teil eines ersten Schlämpestroms (17) gewaschen wird, den man an einem Destillations-Zwischenniveau im Destillationsschritt (5) abnimmt und dem Prozess wieder zuführt, und dass man einen zweiten Schlämpestrom (24) mit höherem Trockenanteil als der erste Schlämpestrom (17) als Bodenprodukt aus dem Destillationsschritt (5) abnimmt und aus dem Prozess abführt.

3. Verfahren nach einem der vorhergehenden Ansprüche, dadurch gekennzeichnet, dass man den hefefreien Feststoffstrom (30) beispielsweise in einem Presschnitt (31) weiter konzentriert, wobei er in einen Konzentratstrom (33) und einen Flüssigkeitsstrom (32) aufgetrennt wird, der in den Prozesskreis, vorzugsweise zum Fermenter (7), zurückgeführt wird.

4. Verfahren nach einem der vorhergehenden Ansprüche, dadurch gekennzeichnet, dass ein erster Schlämpestrom (17) an einem Zwischenniveau im Destillationsschritt (5) abgenommen und in den Prozess zurückgeführt wird und dass ein zweiter Schlämpestrom (24) mit einem Trockenanteil von mehr als 35 Gew.-% als Bodenprodukt vom Destillationsschritt (5) abgenommen und aus dem Prozess entfernt wird.

5. Verfahren nach einem der vorhergehenden Ansprüche, dadurch gekennzeichnet, dass der hefefreie Feststoff (30) oder das Konzentrat (33) in einem Trockenschritt (34) zusammen mit einer als Bodenprodukt vom Destillationsschritt (5) abgenommenen Schlämpe (24) zu einem als Futter nutzbaren geeigneten Trockenprodukt (35) getrocknet wird.

6. Verfahren nach Anspruch 5, dadurch gekennzeichnet, dass der im Trockenschritt (34) erzeugte Wasserdampf gegebenenfalls nach Thermokompression im Prozesskreis als Wärmeträger verwendet wird, beispielsweise als Wärmeträger im Destillationsschritt (5) und/oder im Substrat-Vorbereitungsschritt (2).

## Revendications

1. Un procédé pour la production d'éthanol, par fermentation continue d'un substrat (6) contenant de l'hydrate de carbone d'une matière première contenant des substances fermentables ainsi que des substaces nonfermentables solubles solubles et solides, dans lequel le substrat, après avoir été facultativement traité dans une étape de préparation du substrat (2) comprenant par exemple une hydrolyse de la matière première, est fermenté dans un fermenteur (7), constitué d'un circuit de procédé continu comprenant le fermenteur (7), une étape de séparation de la levure (13) et une étape de distillation (5), un courant (9) de liqueur de fermentation est extrait en continu du fermenteur (7) et séparé, dans ladite étape de séparation de levure (13), en un courant concentré de levure (14), lequel est renvoyé en continu en recirculation dans le fermenteur pour maintenir dans le fermenteur une concentration de cellules de levure correspondant à une certaine productivité d'éthanol, et en un courant pratiquement exempt de levure (15), ledit courant exempt de levure (15) est séparé en continu, dans ladite étape de distillation, en un courant enrichi en éthanol (19a), qui est extrait du circuit de procédé, et en au moins un courant de distillerie (24, 17), une partie dudit courant de distillerie (17) étant envoyée en recirculation continue au fermenteur et/ou à ladite étape de préparation du substrat, et la partie restante dudit courant de distillerie (24) étant évacuée du circuit de procédé, caractérisé en ce que le substrat introduit dans le fermenteur contient ladite substance solide, et que ledit courant de liqueur de fermentation (9) est séparé en un courant (11) enrichi en substance solide et en un courant appauvri en solid (12), lequel est introduit dans ladite étape de séparation de la levure (13), que ledit courant (11) enrichi en substance solide est lavé par un courant de distillerie (26) qui est renvoyé en recirculation dans le circuit de procédé après avoir été refroidi, par exemple par échange de chaleur avec ledit courant exempt de levure (15), à une température non dangereuse pour les cellules de levure qui restent dans ledit courant enrichi en substance solide, et/ou avec une partie de l'eau nécessaire (37) entrant dans le circuit de procédé, après quoi la substance solide lavée est séparée par exemple, dans une étape de filtration (25), en un courant (30) de substances solides pratiquement exemptes de levure, qui est évacué, et en un courant de liqueur de lavage (29), qui est renvoyé au fermenteur.

2. Un procédé selon la revendication 1, caractérisé en ce que ledit courant enrichi en substances solides (11) est lavé avec au moins une partie d'un premier courant de distillerie (17), qui est extrait d'un niveau de distillation intermédiaire dans l'étape de distillation (5) et renvoyé en recirculation dans ledit circuit de procédé, et qu'un deuxième courant de distillerie (24) ayant une teneur plus élevée en substances sèches que ledit premier courant de distillerie (17) est extrait de ladite étape de distillation (5) sous la forme d'un produit de fond, et éliminé dudit circuit de procédé.

3. Un procédé selon l'une quelconque des revendications précédentes caractérisé en ce que ledit courant (30) de substances solides, exempt de levure, est soumis à une nouvelle concentration, par exemple dans une étape de pressage (31), ce qui crée sa séparation en un courant concentré (33) et un courant de liqueur (32) qui est renvoyé audit circuit de procédé, convenablement au fermenteur (7).

4. Un procédé selon l'une quelconque des revendications précédentes caractérisé en ce qu'un premier courant de distillerie (17) est soutiré à un niveau de distillation intermédiaire dans l'étape de distillation (5) et renvoyé en recirculation dans ledit procédé, et qu'un deuxième courant de distillerie (24) ayant une teneur en substances sèches non supérieure à 35% en poids est soutiré comme produit de fond de ladite étape de distillation (5) et éliminé dudit circuit de procédé.

5. Un procédé selon l'une quelconque des revendications précédentes caractérisé en ce que ledit courant (30) de substances solides exemptes de levure ou ledit courant concentré (33) est séché dans une étape de séchage (34) en même temps qu'un courant de distillerie (24) soutiré en tant que produit de fond dans l'étape de distillation (5) pour obtenir un produit séché (35) pouvant être utilisé comme produit alimentaire.

6. Un procédé selon la revendication 5, caractérisé en ce que le courant produit dans l'étape de séchage (34) est utilisé, après thermocompression facultative, comme fluide caloporteur dans ledit circuit de procédé, par exemple comme fluide caloporteur dans l'étape de distillation (5) et/ou dans l'étape de préparation du substrat (2).